# EUROPEAN PATENT SPECIFICATION

(11) **EP 1 805 269 B1**
(45) Date of publication and mention of the grant of the patent: **26.03.2008**
(21) Application number: 05798624.2
(22) Date of filing: 20.10.2005
(51) Int. Cl.: C09D 13/00, A61K 6/00

(54) **PROCESS FOR THE PRODUCTION OF PIGMENT-BEARING LEADS**
VERFAHREN ZUR HERSTELLUNG VON PIGMENTHALTIGEN MINEN
PROCEDE DE PRODUCTION DE MINES CONTENANT DES PIGMENTS

(30) Priority: 22.10.2004 DE 102004051618
(43) Date of publication of application: 11.07.2007
(73) Proprietor: Schwan-STABILO Cosmetics GmbH & Co. KG, 90562 Heroldsberg (DE)
(72) Inventor: PEUKERT, Jürgen, 91257 Pegnitz (DE); PINZER, Reinhard, 91220 Schnaittach (DE); SPROGAR, Christian, 91088 Bubenreuth (DE)
(74) Representative: Leissler-Gerstl, Gabriele
(86) International application number: PCT/EP2005/011312
(87) International publication number: WO 2006/045539

(56) References cited:
- DE-A1- 3 103 128
- US-A- 4 714 085

## Description

The invention concerns a process for the production of pigment-bearing and/or effect substance-bearing leads and leads which can be produced with that process for cosmetic pencils. The invention also concerns the use of such leads.

Pigment-bearing and/or effect substance-bearing leads are dry leads which are also referred to as powder leads, and can be used either in lead pencils or rotary pencils. For lead pencils, the lead is placed in a casing blank which is provided for same and which is then subjected to further processing in per se known manner to form a lead pencil. For a rotary pencil, the lead is fitted into a rotary mechanism. In both cases the pencil serves to apply aesthetic and coloured accents to skin and mucous membrane, in particular to the skin of the face, lips, eyelids and in the region of the eyes. In particular decorative cosmetics are considered as the main areas of use for pigmented powder leads. Pencils with powder leads are used in particular for eyeshadow, lipliner, eyeliner and eyebrow pencils. A further area of use are rouge pencils and concealer pencils. The leads used for those pencils are to be easy and pleasant to apply without scratching the sensitive skin, they are to adhere well after application to the place where they were applied and they are to be as transfer-resistant as possible, that is to say they are not to come out on to other surfaces and articles and they are not to bleed out of the region in which they were applied.

Those kinds of dry powder lead and various processes for the production thereof are known. In the case of the processes known from the state of the art, powder leads can either be produced by pressing a suitable and appropriately pretreated material into a shape or by extruding a material which is made into a paste in a suitable shape, cutting it to length and then drying it. For the sake of better handling those lead portions can be shrunk into a shrink tube. It is also possible to produce a suitable lead material using calcium sulphate hemihydrate (gypsum), make the material into a paste with water, and extrude it before it hardens. The last-mentioned process is less advantageous because the gypsum then very quickly and irreversibly hardens and then makes the material unusable.

Powder leads produced using the specified processes are generally, glued into wood and are then offered in pencil form, but it is also known for such powder leads to be enclosed with a film sheathing which can be at least partially torn away, comprising paper, plastic or metal film or the like. Finally, there were also attempts to present such powder leads in a clamping casing sleeve, similarly to a lead pencil extension device.

References to the specified processes are to be found inter alia in DE-A 31 03 128, EP-A 0 057 266, DE-A 33 02 803, DE-A 33 47 756, DE-A 34 17 115 or DE-A 34 37 989. Processes are also known for the production of marbled powder leads and references thereto are to be found in US No 4 714 085. DE 31 03 128 and DE 35 35 999 describe processes for encasing a powder lead with a shrink tube. Leads of the specified kind were fitted into a suitable, sharpenable casing - any irregularities in the composition could be easily removed by sharpening. From the point of view of a customer however the operation of sharpening a cosmetic pencil is ultimately unsatisfactory as in that case a part of the makeup material is always unused and wasted.

Attempts were therefore undertaken to use leads of the specified kind in rotary mechanisms in order to use them in the manner known from lipsticks. The lead portions necessary for that purpose can be produced in the above-indicated manner and if necessary also shrunk in position. For that purpose the lead material is suitably made into a paste, extruded, dried and the resulting lead portion is then subjected to further processing. For extrusion of the material, particularly if the wish is to produce extrusions of a diameter in the range of 6 to 12 mm, high pressures in the range of 30 to 80 MPa are required. In that case, different flow speeds can occur within the lead material to be extruded, particularly when pearl pigments in flake form or also other flake-form, non-colouring ingredients such as for example transparent mica which is not coloured with metal oxides, synthetic mica, fine glass flakes or the like are used. Those pearl pigments and other flake-form ingredients can in that case also be oriented in a given manner and preferred direction, in accordance with the 'law of least resistance', and thereby provide zones which are structured in different ways, comprising the flake-form substances which are 'oriented in the preferred direction' and the other solid substances, within the lead. As moreover the flow speed of the material in the edge region of the tools necessary for the extrusion operation is lower than in the interior of the extrusion, in that way the edge regions can become very hard and support the interior, which then in itself is soft, of a portion of lead. Upon being applied to the skin, those hard edge zones of the lead portions produce a very unpleasant scratchy feel on the skin.

In addition those leads produce a non-homogeneous result as, depending on the respective viewing angle, apparently completely different colour effects occur by virtue of the preferred direction which is involved in terms of colour reflection and thus lead the lady consumer to believe that there is an apparently different and non-homogeneous material involved.

It is therefore necessary to seek such limit conditions which afford a visually homogeneous lead material, provide leads which as far as possible involve pearl flakes of different orientation over all regions and thus present a uniform coloration with a readily perceptible, intensive pearl sheen.

Therefore the object of the invention was to provide a lead material which has improved flow properties upon extrusion and which can be so extruded that a homogeneous lead is obtained. A further object of the invention was to provide leads which, in spite of a relatively large diameter for the lead extrusions, allow low pressures in the extrusion operation. A further object of the invention was to provide leads having a uniform surface, which allow lines to be drawn and/or permit application over an area and which at the same time have a pleasant feel on the skin.

That object is attained with a process as defined in claim 1.

In accordance with the invention there is provided a process for the production of pigment-bearing and/or effect substance-bearing leads, wherein pigments and/or effect substances are mixed with fillers and further lead ingredients, the resulting material is homogenised and then extruded to provide extrusions of predetermined diameter, wherein then the extruded portions are dried optionally after adjustment of the extrusion length to a constant weight, wherein at least a proportion of the pigment and/or effect material is brought into contact with a polysaccharide solution and an aqueous solution of a precipitation agent prior to admixing of the further ingredients, the mixture is dried and then optionally ground.

In an embodiment of the process according to the invention the following steps are carried out:
a) at least a part of the pigments and/or effect substances is mixed with a polysaccharide solution and an aqueous solution of a precipitation agent,
b) then the mixture is dried to a residual moisture content of 20 to 80%,
c) optionally then a further proportion of the pigment and effect substances is mixed with fillers, binding agents, thickening agents, lubricants, tensides and/or further ingredients and/or mixtures thereof,
d) the mixture from step a) and the mixture from step c) are combined, possibly a liquid is added and then homogenised by kneading,
e) the homogenised mixture is extruded to form extrusions of predetermined diameter, and
f) the extruded portions are dried to a constant weight optionally after adjustment of the extrusion length.

It was surprisingly found that, when a process with specified steps a) to f) is used, leads with surprisingly advantageous properties are obtained. In particular the process according to the invention provides that the lead materials are extruded to afford very homogeneous leads which permit uniform application and which even after prolonged use do not form hard or unpleasant edges.

It was found that this particular homogeneity of the lead material can be achieved when the pigments and/or effect substances are intimately mixed prior to mixing with the other constituents with a polysaccharide. That intimate mixture is achieved by the particulate substances being brought into contact with a generally aqueous solution of a polysaccharide and a solution, generally also aqueous, of a precipitation agent for the saccharide. The sequence in which the two solutions are added is in that respect not critical but preferably in accordance with the invention firstly the particulate material is mixed with a solution of the precipitation agent, for example by kneading, and then a solution of a polysaccharide is added. In that situation the polysaccharide is precipitated in very fine form and can exert its positive effect on the mixture.

The polysaccharides suitable for this process are those natural and synthetic polysaccharides which can be precipitated with a precipitation agent. Alginic acid and pectins are to be mentioned here as particularly suitable. The term "pectins" when used in this application shall include all known types of pectins and pectin derivatives in high methoxylated, low methoxylated and free form, natural products and synthetic ones. Examples of pectins that are products such as pectin, pectic acid, pectinic acid, and galacturonic acid and the esters thereof. Those polysaccharides are dissolved in a suitable amount of water, in which respect the amount itself is not critical but must be sufficient to dissolve the polysaccharide. An excessively high proportion of water is detrimental as the water has to be removed in the later stages of the process.

Suitable precipitation agents for polysaccharides are sequestering agents which, with acid or hydroxy groups, form complexes which precipitate. For polysaccharides which have acid groups, it is possible to use sequestering ions, in particular divalent and polyvalent metal irons. Calcium and magnesium irons and in particular calcium salts have been found to be particularly suitable. In accordance with the invention calcium chloride is particularly preferably used in aqueous solution as a precipitation agent. The amount of precipitation agents is adjusted depending on the respective amount of the polysaccharide to be precipitated and can be easily calculated by the man skilled in the art.

In a preferred embodiment the pigments and/or effect substances are mixed with the aqueous solution of the precipitation agent and upon addition of the aqueous solution of the polysaccharide, the polysaccharide is caused to precipitate in finely divided form. The water is then substantially removed from that mixture, which can be effected using conventional means, for example by filtration, suction removal, drying and so forth. Preferably the water content of the resulting mixture is adjusted to a residual moisture content of 80%, preferably 50% or less. It is particularly preferred for the mixture obtained also to be ground.

The further ingredients which are intended for the lead material are then added to the pigment/polysaccharide mixture prepared in that way and the resulting material is preferably subjected to a homogenisation operation to ensure uniform mixing.

The material obtained after the mixing or homogenising operation can then be extruded in per se known manner to produce extrusions of predetermined diameter. The extruded portions are then dried, optionally after adjustment of the extrusion length, to a constant weight.

In a preferred embodiment of the process according to the invention a part of the pigments/effect substances can be subjected to the above-described steps a) and b) while a further proportion of the pigments and effect substances is mixed and kneaded with a fluid wetting agent so as to obtain a kneadable material. Then the kneadable material and the material obtained in step b) are mixed and homogenised with the other ingredients. The further procedure is then effected as described above. That variant of the process is particularly suitable if the nature of the pigments and effect substances to be incorporated into the lead is different, that is to say if the pigments and effect substances to be added differ greatly in their hydrophilic or hydrophobic property.

In accordance with a further embodiment of the invention therefore a part of the pigments and effect substances is mixed with a fluid wetting agent, thus resulting in a kneadable mixture to which the further ingredients are added, optionally the resulting material is homogenised and extruded to provide extrusions of predetermined diameter and then the extruded portions are dried to a constant weight, optionally after adjustment of the extrusion length.

The variants of the process according to the invention are suitable for all pigments and effect substances which are to be processed to provide powder leads, as are used in cosmetics. The terms pigments and effect substances are used here in the broadest sense to denote all substances which are incorporated into leads to produce a coloured, fluorescent, iridescent, light-reflecting impression and colourless pigments, particularly if they produce a special effect, are also embraced by those terms. Accordingly the term pigment in accordance with the present invention is intended to denote not only the conventional inorganic pigments but generally colourless, white or coloured inorganic or organic pigments, but also colour lakes, pearlescent agents and so-called 'light diffusing pigments' or 'LDP' and lakes of organic dyes. Metal powders and white or colourless powders such as mica, kaolin or talc can also be embraced thereby. The following can be mentioned by way of example for inorganic pigments, for example yellow, black or red iron oxides, titanium dioxide, zirconium oxide, cerium oxide, ultramarine, oxides of zinc and chromium, for example chromium oxide green, chromium hydroxide green, carbon black; organic pigments, lakes of organic pigments, carmine, flake-form metal powders such as passivated aluminium, brass, bronze, copper, silver or gold; mica, micas coated with metal oxides, for example with titanium dioxide, iron oxides, chromium oxide, chromium hydroxide; flake-form preparations based on silicon dioxide, aluminium oxide or glass, which can possibly also be coated with metal oxides, titanium dioxide, iron oxide, chromium oxide, chromium hydroxide and mixtures thereof. In accordance with the invention the term effect substances is used to denote all agents producing an aesthetic impression, for example luminescent, fluorescent, phosphorescent, iridescent, mother-of-pearl-like and thermochromic substances, neon pigments, luminous pigments, interference pigments, metal flakes or spangles, colour-coated aluminium flakes or PET films, holographic elements, pearlescent agents and also UV-active dyes which in daylight and/or artificial lighting and/or with black light produce an effect. The terms pigments and effect substances overlap and are not deemed to be mutually exclusive.

In order to produce leads from pigments and/or effect substances, it is necessary to use binding agents, optionally fillers and thickening agents. All binding agents and fillers which are generally known for the production of cosmetic leads can be considered for production of the leads according to the invention. The binding agents and fillers serve to influence consistency and are used in dependence on the consistency desired for the lead material or the finished lead. The fillers or thickening agents for cosmetic leads that can be considered are for example natural gums and polymers such as xanthan gum, tragacanth and cellulose derivatives, synthetic polymers such as polyesters and polyolefins, inorganic substances such as clays, silicic acid, talc, kaolin, bentonite, hectorite, montmorrillonite, smectite, magnesium-aluminium-silicate, or metal soaps such as aluminium, magnesium, calcium or zinc stearate.

Preferably an inert wetting agent is used as the fluid wetting agent. The term 'inert' in accordance with the present invention denotes such a wetting agent which neither dissolves nor reacts with the pigments and/or effect substances and also binding agents and fillers and is also not absorbed. The inert agent serves to cause the particulate constituents - in particular pigments and effect substances - to slip past each other in the mixing operation without clinging to each other so that a homogeneous material can be formed. Suitable fluid wetting agents are in particular silicon-bearing compounds, for example siloxanes, and fluid hydrocarbons.

Preferred silicon-bearing wetting agents are compounds with repetitive R₂SiO_{2/2} units, wherein R is C₁-C₆ alkyl, cycloalkyl or aryl, preferably phenyl. Particularly preferred are those compounds with repetitive dimethylsiloxane units which are available in great varieties. Short-chain linear siloxanes and cyclic siloxanes which are also commercially available have proven to be particularly suitable. For example silicones made up from dimethylsiloxane units are available by the name dimethicone for linear siloxanes and cyclomethicone for cyclic siloxanes. Silicones made up for example from dimethylsiloxane units are commercially available under the name dimethicone for linear siloxanes and cyclomethicone for cyclic siloxanes. The process and the leads of the present invention particularly preferably use linear dimethicones with 3 to 10 siloxane units, in particular 4 to 8 siloxane units, and cyclomethicones with 3 to 8, in particular 4 to 6 dimethylsiloxane units. The latter are commercially available under the names octamethylcyclotetrasiloxane, decamethylcyclopentasiloxane, and dodecamethylcyclohexasiloxane. Mixtures of those siloxanes are also suitable, which are also commercially available.

In particular paraffins and isoparaffins can be mentioned as fluid hydrocarbons. C₁₁-C₁₂ isoparaffin is a particularly suitable example. Short-chain monovalent alcohols such as ethanol, propan-1-ol, propan-2-ol, esters such as for example ethyl acetate, ketones such as for example acetone or methylethylketone or also mixtures of those liquids are also suitable so long as they are inert (as defined above) for the respective particulate constituents.

A further ingredient which is used according to the invention and which is preferably employed for the lead material according to the invention is a lubricant which adjusts the slip capability of the lead material during the extrusion operation. Both liquid and also solid agents are suitable as the lubricant, in which respect lubricants can act as a kind of lubricating agent while solid lubricants can perform the function of a ball bearing.

The materials usually employed for cosmetic materials can be used as lubricants for the lead according to the invention. Solid lubricants for the lead material according to the invention are inter alia fatty acid salts and boron nitrides in finely divided form such as for example fatty acid salts and boron nitride.

It has been found that particularly good results are achieved if fluorinated or phenylated siloxanes are added to the material as lubricant. Fluorinated and phenylated silicones or siloxanes are known per se and are commercially available. As an example mention may be made for example of perfluorononyldimethicone or methylphenyldimethicone. Copolymers of various siloxane units are also suitable for that purpose. As an example mention may be made of dimethicone/vinyldimethicone copolymer. Ethoxylated and/or propoxylated glycerides or glycerine partial esters have also proven satisfactory as a lubricant. PEG-20 glyceryl stearate can be mentioned here as a particularly suitable example. Mixtures of the specified constituents are also appropriate.

Tensides can also contribute to the lubricating action and are therefore used in preferred embodiments. Preferably the tensides are added prior to the extrusion operation. Examples of suitable tensides are the cationic, anionic, non-ionic or amphoteric tensides which are usually used for cosmetic materials or mixtures thereof, preferably glycerides and glycerine partial fatty acid esters, but also esters of long-chain fatty acids and long-chain alcohols and further classes of compounds which are well-known to the man skilled in the art can be considered depending on the nature of the other ingredients.

The fillers that can be considered for cosmetic leads are in particular talc, kaolin and mica which at the same time can also act as pigments and/or effect substances.

Depending on the respective composition involved preserving agents can also be added to the leads. When dealing with water-free lead materials that is generally not necessary, while the usual ones are employed for water-bearing materials. Further usual ingredients for leads are antioxidants which improve storability and durability. An example in that respect is tocopherol derivatives. It is optionally also possible to use fragrances and aroma substances.

Frequently synthetic, mineral, animal or vegetable waxes are also used for adjusting consistency, such as paraffin, beeswax, carnauba and candelilla wax; oil raw materials such as hydrogenated vegetable oil or triglycerides, for example caprylic/capric triglycerides, and fats.

To improve workability the materials can contain moistening agents for keeping them moist, in the usual amounts. Examples in that respect are glycerine, 1,2-propylene glycol and 1,2-hexylene glycol.

To adjust consistency and to impart specific properties, synthetic or natural polymers are frequently also added to leads, which increase breaking and flexural strength, contribute to particularly good delivery properties and afford a pleasant feel on the skin. Examples of such polymers are carrageenan, tragacanth, alginate, carboxymethylcellulose, hydroxyethylcellulose, hydroxymethylcellulose as natural polymers and acrylates, methacrylates, polyurethanes, polyetherurethanes, polyesters and polyethers as synthetic polymers. Mixtures of individual natural polymers, individual synthetic polymers or natural and synthetic polymers can be used. The polymers are appropriately used in the form of aqueous dispersions to facilitate homogeneous distribution.

The leads according to the invention can be processed to provide concealer pencils and in addition to the constituents which have rather an aesthetic effect can also have therapeutic agents such as panthenol, salicylic acid or levulinic acid derivatives such as phenylethyl- and benzylesters, or other ingredients suitable for treating skin blemishes.

If a wetting agent is used its proportion is preferably in a range of 1 to 10% by weight, in particular 2 to 6% by weight, with respect to the weight of the lead material prior to extrusion. Mixing of pigments and effect substances with the wetting agent can be effect in a period of a few seconds to hours, for example within 10 seconds to about 60 minutes.

The further ingredients are then added to the kneadable material obtained in step a) and b) respectively of the process according to the invention and the resulting material is then optionally homogenised. Homogenisation is preferred in order to obtain a uniform material which results in a lead which is satisfactory aesthetically and in terms of its properties of use. Homogenisation is usually achieved in a period of a few seconds up to 120 minutes depending on the nature of the mixing apparatus used and the properties of the lead material. A period of 10 to about 300 seconds is generally sufficient.

The fillers and binding agents and optionally further lead ingredients which are used if required and optionally the rest of the pigments and/or effect substances are added to the kneadable material and intensively mixed. The ingredients involved in step d) can also be mixed with a fluid wetting agent to simplify the mixing procedure. For that purpose it is possible to use the same wetting agents as stated for step a), different agents therefrom and/or mixtures thereof.

Mixing of the constituents is effected in per se known mixing apparatuses, for example vibration mixers or ploughshare mixers. Homogenisation can then be effected in apparatuses which are also known per se and the man skilled in the art can select the homogeniser which is particularly suitable for the respective purpose.

The material obtained after step d) has flow properties which permit extrusion at lower pressures than is possible with the materials known in the state of the art. As a result the material also flows uniformly through the extrusion tool and supplies very uniform leads having mechanically advantageous properties. The homogenised material is therefore passed through an extrusion tool and extruded to form extrusions of predetermined diameter. The diameter of the extrusions depends on the respective purpose of use and can be adjusted by the man skilled in the art in per se known manner. The diameter depends on the one hand on the intended sleeve casing or rotary mechanism in which the lead is later to be fitted and on the other hand on the proportion of shrinkage which occurs due to subsequent drying. Usually the lead material is extruded to afford extrusions of a diameter in the range of 4 to 20 mm, in particular 6 to 12 mm. When dealing with leads of smaller diameter, the strength is generally too low to be able to use them in rotary mechanisms. Leads of a diameter larger than 20 mm are not preferred for the intended use. Leads of a diameter in the range of 6 to 12 mm have proven to be particularly suitable for the usual lead pencils and rotary mechanisms as they combine a particularly advantageous combination of breaking and flexural strength with application properties and delivery properties.

After the extrusion operation the extruded portions are usually cut to a desired extrusion length which is suitable for being laid in casing sleeves or fitted into rotary mechanisms. The corresponding pencil length depends on the respective casing sleeve or rotary mechanism and is known to the man skilled in the art. A suitable extrusion length is in the range of 20 to 100, preferably 30 to 100 mm.

The extrusions are then dried to a constant weight. That drying operation to a constant weight is necessary to be able to manufacture reproducible leads. It has been found that, when carrying out the process according to the invention, leads of reproducible dimensions are obtained so that the process is suitable for lead manufacture. Drying to a constant weight, in particular with two drying sections in the preferred embodiment, provides leads of predeterminable and easily adjustable dimensions.

Extrusion of the lead material is effected in per se known manner in per se known extrusion tools. Tools and processes for same are numerous and are known to the man skilled in the art in this field. More detailed description in that respect is not required.

An essential feature of the process according to the invention is drying to a constant weight. The expression 'drying to a constant weight' is used to mean that the leads are dried until their weight no longer changes at a steady temperature for at least 5 minutes.

To carry out the drying operation, the extrusions which have possibly been cut to length are either exposed to a constant temperature for a sufficient period of time or preferably, they are exposed firstly to a lower temperature and then to a higher temperature, in two steps. If drying is effected at a constant temperature, temperatures in the range of 40 to 80°C have proven to be suitable, which can be applied over a period of 1 to 24 hours. Particularly good properties are obtained for the leads when drying is effected firstly at a lower temperature and then at a higher temperature. In a particularly preferred procedure a vacuum is applied in the first drying section and then heating to a higher temperature is effected without a vacuum. Particularly good properties are achieved if, in the first step, a temperature of about 40 to 80°C is used and a vacuum of up to 80 kPa is applied. That first drying section can last for 5 minutes up to 12 hours and is preferably effected over a period of 10 to 120 minutes. In a second drying section drying is then effected without vacuum at a higher temperature to a constant weight, which can be checked in per se known manner by repeatedly taking samples and determining the weight thereof. Temperatures of the range of 80 to 150°C, in particular 90 to 120°C, have proven to be suitable temperatures for the second drying section. That second drying section is effected until a constant weight is reached, which is normally after 1 to 6 hours. The duration of that second drying treatment is not critical, the only essential point is that a constant weight is reached. Therefore the respectively appropriate drying period varies in dependence on the temperature used.

The advantages of the process according to the invention, that is to say reproducible, aesthetically and mechanically satisfactory leads, are achieved if at least a part of the pigments and/or effect substances is mixed with the fluid wetting agent and kneaded and drying of the leads is effected in the above-specified manner.

A further subject of the invention is leads which can be obtained with the process according to the invention. Those leads are distinguished in relation to known leads in that they have a particularly homogenous and regular structure so that in use the applied colour or the applied effects remain the same.

A further subject of the invention is the use of the leads produced in accordance with the invention for the production of cosmetic pencils, in particular for producing eyeliners, lipliners, concealer and rouge pencils, eyebrow pencils, kohl and eyeshadow pencils.

The invention is further described by the Examples hereinafter.

The invention is to be described with reference to the following Examples, without this being definitive considerations in this respect. The raw materials used are in that respect denoted by the 'INCI names' which are known to the man skilled in the relevant art, and all amounts are stated in percent by weight (% by weight) with respect to the total weight of the extrudable paste.

### Example 1 (eyeshadow)

| | | |
|---|---|---|
| (A 1) | Mica and titanium dioxide | |
| | (C.I.-No. 77019, 77891) | 48.000 |
| | Calcium chloride | 3.500 |
| | Aqua | 48.500 |
| | | |
| (A 2) | Alginic acid | 0.500 |
| | Aqua | 49.500 |
| | | |
| (B) | Yellow iron oxide (C.I.-No. 77492) | 0.750 |
| | Red iron oxide (C.I.-No.77491) | 1.800 |
| | Black iron oxide (C.I.-No.77499) | 0.250 |
| | Dimethicone/vinyldimethicone copolymer | 2.000 |
| | Polyethylene (powder) | 4.500 |
| | Talc | 1.300 |
| | Boron nitride | 3.000 |
| | Kaolin | 2.500 |
| | | |
| (C) | Diethylhexyl sodium sulphosuccinate | 0.500 |
| | PEG-40 hydrogenated castor oil | 0.350 |
| | Phenoxyethanol | 0.500 |
| | Methylparaben | 0.150 |
| | Propylparaben | 0.050 |
| | Aqua | 32.350 |

For production purposes the water from phase (A1) is provided in a tank with propeller agitator, the calcium chloride is dissolved therein and the titanated mica is scattered thereon. Agitation is thereafter effected until a lump-free suspension is produced. In a separate vessel the alginic acid is dissolved in water with slight heating and agitation to about 50°C and the mixture (A2) is added to the mixture (A1). Mixing is continued for about 30 minutes, followed by filtration, washing with a little water, and drying of the residue at 50°C and under a vacuum of about 60 kPa to a constant weight, with granulation of that preparation.

The constituents of phase (B) are mixed in a ploughshare mixer, the preparation from phase (A) is added as a granulate and mixing is effected for about 20 minutes. In the meantime, in a separate vessel, the mixture of the constituents of phase (C) is produced with slight heating - initially without the addition of water - and then the water is added. That mixture of phases (A) and (B) is now transferred into a kneader, the aqueous phase (C) is added and mixing is effected for about 30 minutes until a homogeneous extrudable paste is produced.

The material is now extruded to form extrusions of a diameter of 8 mm and cut to length to provide portions 50 mm in length. Those portions now remain at ambient temperature for about 30 minutes and are then dried at 50°C in a vacuum of 60 kPa. Drying is then effected to a constant weight without vacuum at 110°C. The result obtained is a markedly medium-brown coloured powder lead with a high sheen which can be easily and workably distributed on the skin and enjoys good adhesion.

### Example 2 (lip powder, matt)

| | | |
|---|---|---|
| (A 1) | Mica (C.I.-No. 77019) | 15.000 |
| | Mica and titanium dioxide | |
| | (C.I.-No. 77019, 77891) | 9.000 |
| | Calcium chloride | 1.750 |
| | Aqua | 24.250 |
| | | |
| (A 2) | Pectin | 0.380 |
| | Aqua | 22.000 |
| | | |
| (B) | Red iron oxide (C.I.-No.77491) | 1.150 |
| | Yellow iron oxide (C.I.-No. 77492) | 0.225 |
| | Titanium dioxide (C.I.-No. 77891) | 1.250 |
| | Dimethicone/vinyldimethicone copolymer | 1.000 |
| | Polyethylene (powder) | 2.500 |
| | Boron nitride | 1.750 |
| | Magnesium stearate | 0.800 |
| | Talc | 1.100 |
| | | |
| (C) | Diethylhexyl sodium sulphosuccinate | 0.250 |
| | PEG-40 hydrogenated castor oil | 0.175 |
| | Phenoxyethanol | 0.250 |
| | Methylparaben | 0.075 |
| | Propylparaben | 0.025 |
| | Aqua | 17.070 |

Production is effected similarly to Example 1.

### Comparative Example A (eye shadow)

| | | |
|---|---|---|
| (A) | Mica and titanium dioxide | |
| | (C.I.-No. 77019, 77891) | 28.000 |
| | Chromium hydroxide green (C.I.-No. 77289) | 2.800 |
| | Ultramarines (C.I.-No. 77007) | 0.300 |
| | Polyethylene (powder) | 2.250 |
| | Boron nitride | 1.500 |
| | Magnesium stearate | 1.200 |
| | Talc | 1.650 |
| | Aqua | 40.000 |
| | | |
| (B) | Carbomer | 0.300 |
| | Triethanolamine | 0.085 |
| | Diethylhexyl sodium sulphosuccinate | 0.250 |
| | PEG-40 hydrogenated castor oil | 0.250 |
| | Phenoxyethanol | 0.250 |
| | Methylparaben | 0.075 |
| | Propylparaben | 0.025 |
| | Aqua | 21.070 |

For production purposes the water of phase (A) is provided in a suitable vessel, then the pigments are smoothly stirred under by means of a propeller agitator, the remaining water is provided in a separate vessel and the carbomer is dissolved therein. The preserving agents are dissolved in the two tensides, possibly with slight heating, and that mixture is added to the water phase. The pH-value of the mixture is then adjusted to 6.4 to 6.7, the carbomer gelling and an increase in viscosity of the mixture occurring. The phase (B) is now homogeneously mixed with the phase (A), the high-viscosity paste obtained is cast in moulds or extruded to form lead extrusions and then dried to a constant weight. It is noteworthy in that respect that the products obtained do not have a uniformly coloured surface. Rather the surface changes according to the respective incidence of light. The leads produced by extrusion also have edge regions of differing hardness and are therefore perceived by the lady user as scratchy and unpleasant upon application.

## Claims

1. A process for the production of leads which contain pigments and/or effect substances, wherein pigments and/or effect substances are mixed with fillers and further lead ingredients, the resulting material is homogenised and then extruded to provide extrusions of predetermined diameter, wherein then the extruded portions are dried optionally after adjustment of the extrusion length to a constant weight, wherein at least a proportion of the pigment and/or effect material is brought into contact with a polysaccharide solution and an aqueous solution of a precipitation agent prior to admixing of the further ingredients, the mixture is dried and then optionally ground.

2. A process for the production of leads which contain pigment substances and/or effect substances, wherein
a) at least a part of the pigment substances is mixed with a polysaccharide solution and an aqueous solution of a precipitation agent,
b) then the mixture is dried to a residual moisture content of 20 to 80%,
c) optionally a further proportion of the pigment and effect substances is mixed with fillers, binding agents, thickening agents, lubricants, tensides and/or further ingredients and/or mixtures thereof,
d) the mixture from step a) and the mixture from step c) are combined, possibly a liquid is added and then homogenised by kneading,
e) the homogenised mixture is extruded to form extrusions of predetermined diameter, and
f) the extruded portions are dried to a constant weight optionally after adjustment of the extrusion length.

3. A process according to claim 1 or claim 2 **characterised in that** alginic acid or pectic acid is used as the polysaccharide.

4. A process according to one of the preceding claims **characterised in that** a soluble calcium salt is used as the precipitation agent.

5. A process according to one of the preceding claims **characterised in that** calcium chloride is used as the precipitation agent.

6. A process according to one of the preceding claims **characterised in that** the mixture of step a) is dried to a residual moisture content of 35 to 60% by weight.

7. A process according to one of the preceding claims **characterised in that** the residual moisture content of the mixture of step b) is adjusted by filtration.

8. A process according to one of the preceding claims **characterised in that** the residual moisture content is adjusted by drying at 30 to 60°C.

9. A process according to one of the preceding claims **characterised in that** pigments and effect substances are used in a proportion of 20 to 80% by weight with respect to the weight of the finished lead.

10. A process according to one of the preceding claims **characterised in that** pigments and effect substances are used in a proportion of 40 to 75% by weight with respect to the lead material.

11. A process according to one of the preceding claims **characterised in that** colour pigments, pearl pigments, mica, synthetic mica, transparent mica, mica coloured with metal oxides, fine glass flakes, flakes coloured with fluorescent dyes, mica or mixtures of the aforesaid constituents are used as the pigments and effect substances.

12. A process according to one of the preceding claims **characterised in that** a mixture of pigments and effect substances is used.

13. A process according to one of the preceding claims **characterised in that** solid and/or liquid lubricants are additionally added to the mixture in step a) and/or b).

14. A process according to claim 13 **characterised in that** boron nitride powder is added as the solid lubricant.

15. A process according to claim 13 **characterised in that** fluorinated and/or phenylated siloxanes or silicone copolymers are added as the lubricant.

16. A process according to claim 15 **characterised in that** perfluorononyldimethicone is used as the fluorinated siloxane.

17. A process according to claim 15 **characterised in that** dimethicone/vinyldimethicone copolymer is used as the copolymer.

18. A process according to one of the preceding claims **characterised in that** fillers, thickening agents, binding agents, extrusion aids, tensides, antioxidants, fragrances, aroma substances, therapeutic active substances and/or preserving agents or mixtures of said constituents are added as further ingredients in step b).

19. A process according to one of the preceding claims **characterised in that** wax-like polyethylene, polyethylene powder, carnauba, candelilla wax, perfluoro PG20 glyceryl stearate or mixtures thereof are used as binding agent.

20. A process according to one of the preceding claims **characterised in that** tensides are added to the mixture prior to the extrusion operation.

21. A process according to claim 20 **characterised in that** diethylhexyl sodium sulphosuccinate is used as the tenside.

22. A process according to one of the preceding claims **characterised in that** a polymer dispersion, preferably an aqueous polymer dispersion of acrylates, methacrylates, polyurethanes, polyetherurethanes, polyesters, polyethers and/or mixtures of the aforesaid substances is additionally added to the mixture.

23. A process according to one of the preceding claims **characterised in that** after the extrusion operation the extrusions are cut to a predetermined length.

24. A process according to one of the preceding claims **characterised in that** the extruded portions or the extrusions which have been cut to length are dried in vacuum or at a slightly elevated temperature of between 40 and 70°C.

25. A process according to one of the preceding claims **characterised in that** the extrusions are dried by a procedure whereby drying is firstly effected for 5 minutes to 24 hours at 40 to 70°C and thereafter drying is effected in a period of 10 to 24 hours at a temperature of 80 to 160°C.

26. A process according to claim 25 **characterised in that** drying is effected for 5 minutes to 4 hours at a temperature of between 40 and 70°C and then drying is effected for 1 to 6 hours at 90 to 120°C.

27. A process according to one of the preceding claims **characterised in that** after step f) the finished lead is encased with a shrink tube.

28. A process according to one of the preceding claims **characterised in that** in a step a1) only a part of the pigments and/or effect agents is treated with polysaccharide and precipitation agent solution and that in a step a2) a further part of the pigments and/or effect substances is mixed with a fluid wetting agent so that a kneadable material is produced, wherein then in step c) the mixtures obtained in step b) and in step a2) are mixed with the further ingredients and then optionally homogenised.

29. A process according to one of the preceding claims **characterised in that** active substances are additionally added to the mixture.

30. A process according to claim 29 **characterised in that** salicylic acid or levulinic acid benzyl or phenethyl ester is used as the active substance.

31. A process according to claim 28 **characterised in that** the fluid wetting agent is a fluid linear or cyclic siloxane or a fluid hydrocarbon.

32. A process according to claim 31 **characterised in that** short-chain linear dimethicones are used as the siloxane.

33. A process according to claim 32 **characterised in that** dimethicones with 2 to 8 dimethylsiloxane units are used as the siloxane.

34. A process according to claim 28 **characterised in that** a mixture of dimethylsiloxanes and dimethylphenylsiloxanes is used as the siloxane.

35. A process according to claim 28 **characterised in that** cyclic siloxanes are used as the siloxane.

36. A process according to claim 35 **characterised in that** a cyclomethicone with 3 to 8 dimethylsiloxane units is used as the cyclic siloxane.

37. A process according to one of claims 28 to 36 **characterised in that** in step a) the siloxane is added in combination with a solvent such as a short-chain monovalent alcohol, an ester, ketone or a mixture thereof.

38. A process according to claim 37 **characterised in that** ethanol, propan-1-ol, propan-2-ol, ethylacetate, acetone, methylethylketone or a mixture of said solvents is added.

39. A pigment-bearing and/or effect substance-bearing lead which can be obtained with a process according to one of claims 1 to 38.

40. A lead according to claim 39 which is of a diameter of 6 to 20 mm.

41. A lead according to claim 40 **characterised in that** it is of a diameter of 5 to 12 mm.

42. A lead according to one of claims 39 to 41 **characterised in that** it is a marbled powder lead.

43. A lead according to one of claims 39 to 42 **characterised in that** the lead additionally contains active substances.

44. A lead according to claim 43 **characterised in that** the lead contains salicylic acid or levulinic acid benzyl or phenethyl ester as the active substance.

45. Use of a lead produced with a process according to one of claims 1 to 38 for the production of cosmetic pencils which are held in a casing sleeve or in a rotary mechanism.

46. Use according to claim 45 for the production of eyeshadow, eyeliner, eyebrow, lip contour, rouge and/or concealer pencils.

## Patentansprüche

1. Verfahren zur Herstellung von Minen, die Pigmente und/oder Effektstoffe enthalten, wobei Pigmente und/oder Effektstoffe mit Füllstoffen und weiteren Mineninhaltsstoffen vermischt werden, die entstehende Masse homogenisiert wird und dann zu Strängen mit vorbestimmtem Durchmesser extrudiert wird, wobei anschließend die extrudierten Stränge gegebenenfalls nach Einstellung der Stranglänge bis zur Gewichtskonstanz getrocknet werden, wobei zumindest ein Anteil des Pigment- und/oder Effektmaterials vor der Zumischung der weiteren Inhaltsstoffe mit einer Polysaccharidlösung und einer wässrigen Lösung eines Fällungsmittels in Kontakt gebracht wird, die Mischung getrocknet und ggf. anschließend vermahlen wird.

2. Verfahren zur Herstellung von Minen, die Pigment- und/oder Effektstoffe enthalten, wobei
a) mindestens ein Teil der Pigmentstoffe mit einer Polysaccharidlösung und einer wässrigen Lösung eines Fällungsmittels versetzt wird,
b) anschließend die Mischung auf eine Restfeuchte von 20 bis 80% getrocknet wird;
c) gegebenenfalls ein weiterer Anteil der Pigment- und Effektstoffe mit Füllstoffen, Bindemitteln, Verdickungsmitteln, Gleitmitteln, Tensiden und/oder weiteren Inhaltsstoffen und/oder Mischungen davon vermischt wird,
d) die Mischung aus Stufe a) und die Mischung aus Stufe c) vereinigt werden, gegebenenfalls eine Flüssigkeit zugesetzt wird und dann durch Verkneten homogenisiert wird,
e) die homogenisierte Mischung extrudiert wird zu Strängen mit vorbestimmtem Durchmesser und
f) die extrudierten Stränge ggf. nach Einstellung der Stranglänge bis zur Gewichtskonstanz getrocknet werden.

3. Verfahren nach Anspruch 1 oder Anspruch 2, **dadurch gekennzeichnet, dass** als Polysaccharid Alginsäure oder Pektinsäure verwendet wird.

4. Verfahren nach einem der vorhergehenden Ansprüche, **dadurch gekennzeichnet, dass** als Fällungsmittel ein lösliches Calciumsalz verwendet wird.

5. Verfahren nach einem der vorhergehenden Ansprüche, **dadurch gekennzeichnet, dass** als Fällungsmittel Calciumchlorid eingesetzt wird.

6. Verfahren nach einem der vorhergehenden Ansprüche, **dadurch gekennzeichnet, dass** die Mischung von Stufe a) bis auf eine Restfeuchte von 35 bis 60 Gew.-% getrocknet wird.

7. Verfahren nach einem der vorhergehenden Ansprüche, **dadurch gekennzeichnet, dass** die Restfeuchte der Mischung von Stufe b) durch Filtration eingestellt wird.

8. Verfahren nach einem der vorhergehenden Ansprüche, **dadurch gekennzeichnet, dass** die Restfeuchte durch Trocknung bei 30 bis 60°C eingestellt wird.

9. Verfahren nach einem der vorhergehenden Ansprüche, **dadurch gekennzeichnet, dass** Pigmente und Effektstoffe in einem Anteil von 20 bis 80 Gew.-% bezogen auf das Gewicht der fertigen Mine verwendet werden.

10. Verfahren nach einem der vorhergehenden Ansprüche, **dadurch gekennzeichnet, dass** Pigmente und Effektstoffe in einem Anteil von 40 bis 75 Gew.-% bezogen auf die Minenmasse eingesetzt werden.

11. Verfahren nach einem der vorhergehenden Ansprüche, **dadurch gekennzeichnet, dass** als Pigmente und Effektstoffe Farbpigmente, Pearlpigmente, Glimmer, synthetischer Glimmer, transparenter Glimmer, mit Metalloxiden gefärbter Glimmer, feine Glasplättchen, mit fluoreszierenden Farben eingefärbte Plättchen, Glimmer oder Mischungen der genannten Bestandteile verwendet werden.

12. Verfahren nach einem der vorhergehenden Ansprüche, **dadurch gekennzeichnet, dass** eine Mischung aus Pigmenten und Effektstoffen verwendet wird.

13. Verfahren nach einem der vorhergehenden Ansprüche, **dadurch gekennzeichnet, dass** der Mischung in Stufe a) und/oder b) zusätzlich feste und/oder flüssige Gleitmittel zugefügt werden.

14. Verfahren nach Anspruch 13, **dadurch gekennzeichnet, dass** als festes Gleitmittel Bornitridpulver zugefügt wird.

15. Verfahren nach Anspruch 13, **dadurch gekennzeichnet, dass** als Gleitmittel fluorierte und/oder phenylierte Siloxane oder Silicon-Copolymere zugemischt wird.

16. Verfahren nach Anspruch 15, **dadurch gekennzeichnet, dass** als fluoriertes Siloxan Perfluorononyldimethicone verwendet wird.

17. Verfahren nach Anspruch 15, **dadurch gekennzeichnet, dass** als Copolymer Dimethicone/Vinyldimethicone-Copolymer verwendet wird.

18. Verfahren nach einem der vorhergehenden Ansprüche, **dadurch gekennzeichnet, dass** als weitere Inhaltsstoffe in Stufe b) Füllstoffe, Verdickungsmittel, Bindemittel, Extrusionshilfsmittel, Tenside, Antioxidantien, Duftstoffe, Aromastoffe, therapeutische Wirkstoffe und/oder Konservierungsmittel oder Mischungen dieser Bestandteile zugegeben werden.

19. Verfahren nach einem der vorhergehenden Ansprüche, **dadurch gekennzeichnet, dass** als Bindemittel wachsartiges Polyethylen, Polyethylenpulver, Carnauba, Candelillawachs, Perfluoro PG20 Glycerylstearat oder Mischungen davon verwendet werden.

20. Verfahren nach einem der vorhergehenden Ansprüche, **dadurch gekennzeichnet, dass** der Mischung vor der Extrusion Tenside zugegeben werden.

21. Verfahren nach Anspruch 20, **dadurch gekennzeichnet, dass** als Tensid Diethylhexylsodiumsulfosuccinat verwendet wird.

22. Verfahren nach einem der vorhergehenden Ansprüche, **dadurch gekennzeichnet, dass** der Mischung zusätzlich eine Polymerdispersion, bevorzugt eine wässrige Polymerdispersion von Acrylaten, Methacrylaten, Polyurethanen, Polyetherurethanen, Polyestern, Polyethern und/oder Mischungen der vorgenannten Substanzen zugefügt wird.

23. Verfahren nach einem der vorhergehenden Ansprüche, **dadurch gekennzeichnet, dass** nach der Extrusion die Stränge zu einer vorbestimmten Länge geschnitten werden.

24. Verfahren nach einem der vorhergehenden Ansprüche, **dadurch gekennzeichnet, dass** die extrudierten Stränge bzw. die zugeschnittenen Stränge im Vakuum oder bei leicht erhöhter Temperatur zwischen 40 und 70°C getrocknet werden.

25. Verfahren nach einem der vorhergehenden Ansprüche, **dadurch gekennzeichnet, dass** das Trocknen der Stränge erfolgt, indem zuerst 5 Minuten bis 24 Stunden bei 40 bis 70°C getrocknet wird und danach in einem Zeitraum von 10 bis 24 Stunden auf eine Temperatur von 80 bis 160°C getrocknet wird.

26. Verfahren nach Anspruch 25, **dadurch gekennzeichnet, dass** 5 Minuten bis 4 Stunden bei einer Temperatur zwischen 40 und 70°C getrocknet wird und anschließend 1 bis 6 Stunden bei 90 bis 120°C getrocknet wird.

27. Verfahren nach einem der vorhergehenden Ansprüche, **dadurch gekennzeichnet, dass** nach Stufe f) die fertige Mine mit einem Schrumpfschlauch umhüllt wird.

28. Verfahren nach einem der vorhergehenden Ansprüche, **dadurch gekennzeichnet, dass** in einer Stufe a1) nur ein Teil der Pigmente und/oder Effektmittel mit Polysaccharid- und Fällungsmittellösung behandelt wird und dass in einer Stufe a2) ein weiterer Teil der Pigmente und/oder Effektstoffe mit einem fließfähigen Benetzungsmittel vermischt wird, so dass eine knetbare Masse entsteht, wobei dann in Stufe c) die in Stufe b) und die in Stufe a2) erhaltenen Mischungen mit den weiteren Inhaltstoffen vermischt werden und anschließend ggf. homogenisiert werden.

29. Verfahren nach einem der vorhergehenden Ansprüche, **dadurch gekennzeichnet, dass** zusätzlich Wirkstoffe der Mischung zugefügt werden.

30. Verfahren nach Anspruch 29 **dadurch gekennzeichnet, dass** als Wirkstoff Salicylsäure oder Lävulinsäurebenzyl- oder -phenethylester eingesetzt wird.

31. Verfahren nach Anspruch 28, **dadurch gekennzeichnet, dass** das fließfähige Benetzungsmittel ein fließfähiges lineares oder cyclisches Siloxan oder ein fließfähiger Kohlenwasserstoff ist.

32. Verfahren nach Anspruch 31, **dadurch gekennzeichnet, dass** als Siloxan kurzkettige lineare Dimethicone verwendet werden.

33. Verfahren nach Anspruch 32, **dadurch gekennzeichnet, dass** als Siloxan Dimethicone mit 2 bis 8 Dimethylsiloxaneinheiten verwendet werden.

34. Verfahren nach Anspruch 28, **dadurch gekennzeichnet, dass** als Siloxan eine Mischung aus Dimethylsiloxanen und Dimethylphenylsiloxanen verwendet wird.

35. Verfahren nach Anspruch 28, **dadurch gekennzeichnet, dass** als Siloxan cyclische Siloxane verwendet werden.

36. Verfahren nach Anspruch 35, **dadurch gekennzeichnet, dass** als cyclisches Siloxan ein Cyclomethicone mit 3 bis 8 Dimethylsiloxaneinheiten verwendet wird.

37. Verfahren nach einem der Ansprüche 28 bis 36, **dadurch gekennzeichnet, dass** in Stufe a) das Siloxan in Kombination mit einem Lösungsmittel, wie einem kurzkettigen einwertigen Alkohol, einem Ester, Keton oder einer Mischung der genannten zugefügt wird.

38. Verfahren nach Anspruch 37, **dadurch gekennzeichnet, dass** Ethanol, 1-Propanol, 2-Propanol, Ethylacetat, Aceton, Methylethylketon oder eine Mischung dieser Lösungsmittel zugefügt wird.

39. Pigment- und/oder effektstoffhaltige Mine erhältlich mit einem Verfahren gemäß einem der Ansprüche 1 bis 38.

40. Mine nach Anspruch 39, die einen Durchmesser von 6 bis 20 mm aufweist.

41. Mine nach Anspruch 40, **dadurch gekennzeichnet, dass** sie einen Durchmesser von 5 bis 12 mm aufweist.

42. Mine nach einem der Ansprüche 39 bis 41, **dadurch gekennzeichnet, dass** es eine marmorierte Pudermine ist.

43. Mine nach einem der Ansprüche 39 bis 42, **dadurch gekennzeichnet, dass** die Mine zusätzlich Wirkstoffe enthält.

44. Mine nach Anspruch 43, **dadurch gekennzeichnet, dass** die Mine als Wirkstoff Salicylsäure oder Lävulinsäurebenzyl- oder -phenethylester enthält.

45. Verwendung einer mit einem Verfahren nach einem der Ansprüche 1 bis 38 hergestellten Mine zur Herstellung von in einer Hülse oder in einer Drehmechanik gehaltenen Kosmetikstiften.

46. Verwendung nach Anspruch 45 zur Herstellung von Lidschatten-, Eyeliner-, Augenbrauen-, Lippenkonturen-, Rouge- und/oder Concealerstiften.

## Revendications

1. Procédé de production de mines qui contiennent des pigments et/ou substances d'effet, dans lequel des pigments et/ou substances d'effet sont mélangés avec des charges et autres ingrédients de mine, le matériau obtenu est homogénéisé et ensuite extrudé pour fournir des extrusions de diamètre prédéterminé, dans lequel les parties extrudées sont ensuite éventuellement séchées après ajustement de la longueur d'extrusion par rapport à un poids constant, dans lequel au moins une proportion du pigment et/ou du matériau d'effet est amenée en contact avec une solution de polysaccharide et une solution aqueuse d'un agent de précipitation avant ajout des autres ingrédients, le mélange est séché et ensuite éventuellement broyé.

2. Procédé de production de mines qui contiennent des substances de pigment et/ou des substances d'effet, dans lequel
a) au moins une partie des substances de pigment est mélangée à une solution de polysaccharide et une solution aqueuse d'un agent de précipitation,
b) le mélange est ensuite séché à une teneur en humidité résiduelle de 20 à 80%,
c) éventuellement, une autre proportion des substances de pigment et active est mélangée à des charges, agents de liaison, agents épaississants, lubrifiants, tensioactifs et/ou autres ingrédients et/ou mélanges de ceux-ci,
d) le mélange de l'étape a) et le mélange de l'étape c) sont combinés, un liquide est éventuellement ajouté et le tout est ensuite homogénéisé par malaxage,
e) le mélange homogénéisé est extrudé pour former des extrusions de diamètre prédéterminé, et
f) les parties extrudées sont séchées à un poids constant éventuellement après ajustement de la longueur d'extrusion.

3. Procédé selon la revendication 1 ou la revendication 2, **caractérisé en ce que** de l'acide alginique ou de l'acide pectique est utilisé en tant que polysaccharide.

4. Procédé selon l'une des revendications précédentes, **caractérisé en ce qu'**un sel de calcium soluble est utilisé en tant qu'agent de précipitation.

5. Procédé selon l'une des revendications précédentes, **caractérisé en ce que** du chlorure de calcium est utilisé en tant qu'agent de précipitation.

6. Procédé selon l'une des revendications précédentes, **caractérisé en ce que** le mélange de l'étape a) est séché à une teneur en humidité résiduelle de 35 à 60 % en poids.

7. Procédé selon l'une des revendications précédentes, **caractérisé en ce que** la teneur en humidité résiduelle du mélange de l'étape b) est ajustée par filtration.

8. Procédé selon l'une des revendications précédentes, **caractérisé en ce que** la teneur en humidité résiduelle est ajustée par séchage à une température de 30 à 60 °C.

9. Procédé selon l'une des revendications précédentes, **caractérisé en ce que** des pigments et substances d'effet sont utilisés dans une proportion de 20 à 80 % en poids par rapport au poids de la mine finie.

10. Procédé selon l'une des revendications précédentes, **caractérisé en ce que** des pigments et substances d'effet sont utilisés dans une proportion de 40 à 75 % en poids par rapport au matériau de la mine.

11. Procédé selon l'une des revendications précédentes, **caractérisé en ce que** des pigments de couleur, pigments de perles, du mica, du mica synthétique, du mica transparent, du mica coloré avec des oxydes métalliques, des flocons de verre fins, des flocons colorés avec des colorants fluorescents, du mica ou des mélanges des constituants susmentionnés sont utilisés en tant que pigments et substances d'effet.

12. Procédé selon l'une des revendications précédentes, **caractérisé en ce qu'**un mélange de pigments et substances d'effet est utilisé.

13. Procédé selon l'une des revendications précédentes, **caractérisé en ce que** des lubrifiants solides et/ou liquides sont en outre ajoutés au mélange dans l'étape a) et/ou b).

14. Procédé selon la revendication 13, **caractérisé en ce que** de la poudre de nitrure de bore est ajoutée en tant que lubrifiant solide.

15. Procédé selon la revendication 13, **caractérisé en ce que** des siloxanes fluorés et/ou phénylés ou des copolymères de silicone sont ajoutés en tant que substance lubrifiante.

16. Procédé selon la revendication 15, **caractérisé en ce que** du perfluorononyldiméthicone est utilisé en tant que siloxane fluoré.

17. Procédé selon la revendication 15, **caractérisé en ce que** du copolymère de diméthicone/vinyldiméthicone est utilisé en tant que copolymère.

18. Procédé selon l'une des revendications précédentes, **caractérisé en ce que** des charges, agents épaississants, agents de liaison, adjuvants d'extrusion, tensioactifs, antioxydants, parfums, substances aromatiques, substances thérapeutiques actives et/ou agents de conservation ou mélanges desdits constituants sont utilisés en tant qu'autres ingrédients dans l'étape b).

19. Processus selon l'une des revendications précédentes, **caractérisé en ce que** du polyéthylène de type cire, de la poudre de polyéthylène, de la cire de carnauba, de la cire de candelilla, du stéarate de glycéryle PG20 perfluoré ou des mélanges de ceux-ci sont utilisés en tant qu'agent de liaison.

20. Procédé selon l'une des revendications précédentes, **caractérisé en ce que** des tensioactifs sont ajoutés au mélange avant l'opération d'extrusion.

21. Procédé selon la revendication 20, **caractérisé en ce que** du sulfosuccinate de sodium et de diéthylhexyle est utilisé en tant que tensioactif.

22. Procédé selon l'une des revendications précédentes, **caractérisé en ce qu'**une dispersion de polymère, de préférence, une dispersion de polymère aqueuse d'acrylates, de méthacrylates, de polyuréthanes, de polyétheruréthanes, de polyesters, de polyéthers et/ou mélanges des substances susmentionnées est en outre ajoutée au mélange.

23. Procédé selon l'une des revendications précédentes, **caractérisé en ce qu'**après l'opération d'extrusion, les extrusions sont découpées à une longueur prédéterminée.

24. Procédé selon l'une des revendications précédentes, **caractérisé en ce que** les parties extrudées ou les extrusions qui ont été découpées à la longueur sont séchées sous vide ou à une température légèrement élevée comprise entre 40 et 70 °C.

25. Procédé selon l'une des revendications précédentes, **caractérisé en ce que** les extrusions sont séchées par une procédure moyennant laquelle le séchage est tout d'abord effectué pendant 5 minutes à 24 heures à une température de 40 à 70 °C et ensuite, le séchage est effectué sur une période de 10 à 24 heures à une température de 80 à 160 °C.

26. Procédé selon la revendication 25, **caractérisé en ce que** le séchage est effectué pendant 5 minutes à 4 heures à une température comprise entre 40 et 70 °C et le séchage est ensuite effectué pendant 1 à 6 heures à 90 à 120 °C.

27. Procédé selon l'une des revendications précédentes, **caractérisé en ce qu'**après l'étape f), la mine finie est revêtue d'une gaine thermorétrécissable.

28. Procédé selon l'une des revendications précédentes, **caractérisé en ce que**, dans une étape a1), seule une partie des pigments et/ou agents d'effet est traitée avec une solution de polysaccharide et d'agent de précipitation et **en ce que** dans une étape a2), une autre partie des pigments et/ou substances d'effet est mélangée avec un agent mouillant fluide de, sorte qu'un matériau malaxable soit produit, dans lequel ensuite, dans l'étape c), les mélanges obtenus dans l'étape b) et dans l'étape a2) sont mélangés avec les autres ingrédients et ensuite éventuellement homogénéisés.

29. Procédé selon l'une des revendications précédentes, **caractérisé en ce que** des substances actives sont en outre ajoutées au mélange.

30. Procédé selon la revendication 29, **caractérisé en ce que** de l'ester benzylique ou phénéthylique d'acide salicylique ou d'acide levulique est utilisé en tant que substance active.

31. Procédé selon la revendication 28, **caractérisé en ce que** l'agent mouillant fluide est un siloxane linéaire ou cyclique fluide ou un hydrocarbure fluide.

32. Procédé selon la revendication 31, **caractérisé en ce que** des diméthicones linéaires à chaîne courte sont utilisées en tant que siloxane.

33. Procédé selon la revendication 32, **caractérisé en ce que** des diméthicones avec 2 à 8 unités de diméthylsiloxane sont utilisées en tant que siloxane.

34. Procédé selon la revendication 28, **caractérisé en ce qu'**un mélange de diméthylsiloxanes et de diméthylphénylsiloxanes est utilisé en tant que siloxane.

35. Procédé selon la revendication 28, **caractérisé en ce que** des siloxanes cycliques sont utilisés en tant que siloxane.

36. Procédé selon la revendication 35, **caractérisé en ce qu'**un cyclométhicone avec 3 à 8 unités de diméthylsiloxane est utilisé en tant que siloxane cyclique.

37. Procédé selon l'une des revendications 28 à 36, **caractérisé en ce que**, dans l'étape a), le siloxane est ajouté en combinaison avec un solvant tel qu'un alcool monovalent à chaîne courte, un ester, une cétone ou un mélange de ceux-ci.

38. Procédé selon la revendication 37, **caractérisé en ce que** de l'éthanol, du propan-1-ol, du propan-2-ol, de l'éthylacétate, de l'acétone, de la méthyléthylcétone ou un mélange desdits solvants est ajouté.

39. Mine contenant un pigment et/ou une substance d'effet qui peut être obtenue à partir d'un procédé selon l'une des revendications 1 à 38.

40. Mine selon la revendication 39 qui a un diamètre de 6 à 20 mm.

41. Mine selon la revendication 40, **caractérisée en ce qu'**elle a un diamètre de 5 à 12 mm.

42. Mine selon l'une des revendications 39 à 41, **caractérisée en ce qu'**elle est une mine de poudre de marbre.

43. Mine selon l'une des revendications 39 à 42, **caractérisée en ce que** la mine contient en outre des substances actives.

44. Mine selon la revendication 43, **caractérisée en ce que** la mine contient un ester benzylique ou phénéthylique d'acide salicylique ou d'acide lévulique en tant que substance active.

45. Utilisation d'une mine produite avec un procédé selon l'une des revendications 1 à 38 pour la production de crayons cosmétiques qui sont contenus dans un manchon de protection ou dans un mécanisme rotatif.

46. Utilisation selon la revendication 45 pour la production de fard à paupières, eyeliner, crayon à sourcils, contour des lèvres, rouge à lèvres et/ou crayon de masquage.
